Europäisches Patentamt

European Patent Office

Office européen des brevets

(19)

(11) Publication number : **0 464 010 A1**

## (12) EUROPEAN PATENT APPLICATION

(21) Application number : **91850139.6**

(22) Date of filing : **24.05.91**

(51) Int. Cl.⁵ : **C12Q 1/70,** C12Q 1/68,
C07H 21/04, // C12P19/34

(30) Priority : **08.06.90 SE 9002060**

(43) Date of publication of application :
**02.01.92 Bulletin 92/01**

(84) Designated Contracting States :
**AT BE CH DE DK ES FR GB GR IT LI LU NL SE**

(71) Applicant : **STATENS
VETERINÄRMEDICINSKA ANSTALT
Box 7073
S-75 007 Uppsala (SE)**

(72) Inventor : **Belák, Sandor
Malma Backe 60
S-756 47 Uppsala (SE)**
Inventor : **Ballagi-Pordány, András
Geijersgatan 52 B
S-752 31 Uppsala (SE)**

(54) **A method of detecting an infection caused by a specific type of virus, primers, probes and a test kit.**

(57) A method of detecting an infection caused by a specific type of virus in a biological specimen, is described. The specimen is treated in a per se known manner for the purification of viral RNA and transcription of a selected RNA sequence to the corresponding cDNA, followed by a first amplification of said cDNA by the polymerase chain reaction (PCR) using a first pair of primers, optionally dilution of the PCR product and optionally a second amplification of said cDNA by PCR, then said type of virus is identified by hybridizing said amplified cDNA with a labeled probe specific for said type of virus and possibly formed hybrides are detected with the aid of the label used. The specific features are that the specific type of virus is bovine viral diarrhea virus (BVDV) and that the first pair of primers, the optional second pair of primers and the probe are all selected from synthetic oligonucleotide sequences corresponding to oligonucleotide sequences located within the gp48 nucleotide region of 1362 to 1880 of the BVDV genome.

Further, new primers, new probes and a test kit for the detection of an infection caused by BVDV are disclosed.

EP 0 464 010 A1

Fig. 1.

A

B

OSLOSS
NEW YORK-1
OREGON
SINGER
BORGEN
NADL

C

D

IBR
BOV.COR.
PARVO
PARAINF.
NCP 303/89
0715
A-1138/69

The present invention relates to a method of detecting an infection caused by a specific type of virus in a biological specimen. More specifically it relates to a method of detecting an infection caused by bovine viral diarrhea virus (BVDV). Additionally the invention relates to new primers, new probes and a test kit for the detection of an infection caused by BVDV.

## BACKGROUND

Bovine viral diarrhea virus (BVDV) is one of the most important pathogens of cattle, causing economic losses of considerable importance throughout the world.

The genome of BVDV is infectious, positive-strand RNA (Diderholm and Dinter, 1966), estimated at 2.9 to $4.4 \times 10^6$ Da in size (Purchio et al., 1983; Renard et al., 1985; Collett et al., 1988)- BVDV is currently classified as a member of the family <u>Togaviridae</u>, genus <u>Pestivirus</u> (Westaway et al., 1985). Propagation of BVDV in cell cultures allows the differentiation of cytopathic and non-cytopathic biotypes (cp-BVDV and noncp-BVDV). Both biotypes are pathogenic for cattle (Bolin et al., 1985).

There are two disease entities caused by BVDV and occurring predominantly in adult calves: the acute bovine virus diarrhea with high morbidity and low mortality, and the acute or chronic form of mucosal disease with low morbidity and high mortality.

Both entities show suggestive clinical symptoms and lesions. The virus is spread from animals that carry a persistent, latent infection but are usually seronegative. These animals are the offsprings of cows in which, after a primary infection, a transplacental spread occurred during pregnancy. Depending upon the stage of pregnancy, the transplacental infection may result in abortion or stillbirth, in malformation or - immunotolerance. Immunotolerant calves are the virus carriers and extretors but may, after a recurrent infection, develop mucosal disease (Brownlie, 1983).

BVDV also is a frequent contaminant in fetal calf serum, a commonly used component in cell culture systems. This infection may lead to the BVDV contamination of biological products, e.g. vaccines (Baker, 1987).

Due to the obvious economic impact of BVDV infection, attempts are made world-wide to introduce effective control measures. The aim of these is to break the cycle of transmission by identifying and eliminating the sources of infection.

The conventional virus diagnosis of BVDV infection has been based on the direct demonstration of the virus in clinical specimens or on indirect detection by assessment of specific antibody response. Virus isolation as well as the techniques of immunohistochemistry are the most common methods of direct detection, whereas the indirect detection is assessed by various immunoassays.

The present methods are either insensitive or are unsuitable for large-scale screening. Sensitive and novel approaches are needed to trace the spread and circulation of BVDV as well as to study the pathogenesis of the disease.

In recent years efforts have been made to develop direct methods based on the demonstration of the BVDV RNA by nucleic acid hybridization (Renard et al., 1985; Potgieter and Brock, 1989).

The present invention relies on the method of polymerase chain reaction (PCR) using new primers for the detection of the BVDV genome in specimens. This already wellknown method makes it possible to amplify selected gene sequences to amounts that allow their detection and identification. The detection and identification are performed in the present invention by hybridization using a new probe.

The experience we gained on some viral diseases show that the PCR is several orders of magnitude more sensitive than the direct hybridization assays (Belák et al., 1989; Ballagi-Pordány et al., 1990).

## DESCRIPTION OF THE INVENTION

One aspect of the invention is directed to a method of detecting an infection caused by a specific type of virus in a biological specimen, whereby said specimen is treated in a per se known manner for the purification of viral RNA and transcription of a selected RNA sequence to the corresponding cDNA, followed by a first amplification of said cDNA by the polymerase chain reaction (PCR) using a first pair of primers, optionally dilution of the PCR product and optionally a second amplification of said cDNA by PCR, then said type of virus is identified by hybridizing said amplified cDNA with a labeled probe specific for said type of virus and possibly formed hybrides are detected with the aid of the label used. The specific type of virus is bovine viral diarrhea virus (BVDV) and the first pair of primers, the optional second pair of primers and the probe are all selected from synthetic oligonucleotide sequences corresponding to oligonucleotide sequences located within the gp48 nucleotide region of 1362 to 1880 of the BVDV genome. The nucleotide region of 1362 to 1880 of the BVDV genome is found in the gp48 region of the virus, and the numbering used is the one published by Collett et al. in Virology <u>165</u>, 191-199 (1988).

Examples of biological specimens which have been used in the method of the invention are lungs, spleen, placenta and serum from cows. A suitable lable for the probe is biotin-16-dUTP or digoxigenin-16-dUTP.

In an embodiment of the invention the first pair of primers consist of 19 to 23 nucleotides each, and the second pair of primers consist of 27 to 32 nucleotides each. The number of nucleotides in each of the second pair of primers is preferably at least 8 more than in each of the first pair of primers, but the primer sizes may optionally be the same in the first pair of primers and in the second pair of primers.

In a preferred embodiment of the invention the primers are selected from the group consisting of

```
GGTATGATGG ATGCAAGTGA G
AAGCAGCGTA TGCTCCAAAC C
ACTTCAACGC CATGAGTGGA ACAAGCATGG
CTTTTTTCCT AGTATCCCGA GCTGCTTGCC.
```

The probe used in the method of the invention preferably comprises at least 100 nucleotides. In a preferred embodiment of the invention the probe used is

```
GTCATGAATA GAACCCAAGC CAATCTCACT
GAGGGACAAC CACCAAGGGA GTGCGCAGTC
ACTTGTAGGT ATGATAGGGC TAGTGACTTA
AACGTGGTAA CACAAGCTAG AGATAGCCCC
ACACCCTTAA CAGGTTGCAA GAAAGGAAAG
AACTTCTCCT TTGCAGGGAT ATTGATGCGG
GGCCCCTGCA ACTTTGAAAT AGCTGCAAGT
GATGTATTAT TCAAAGAACA TGAACGGATT
AGTATGTTCC AGGATACCAC TCTTTACCTT
GTTGACGGGT TGACCAACTC CTTAGAAGGT
GCCAGACAAG GA.
```

Another aspect of the invention is directed to a primer selected from synthetic oligonucleotide sequences corresponding to oligonucleotide sequences located within the gp48 nucleotide region of 1362 to 1880 of the BVDV genome. In the field of genetic engineering primers of the size 15-40 nucleotides are commonly used. In an embodiment of this aspect of the invention the primer is selected from primers consisting of 19 to 32 nucleotides. In a preferred embodiment of the invention the primer is selected from the group consisting of

```
GGTATGATGG ATGCAAGTGA G
AAGCAGCGTA TGCTCCAAAC C
ACTTCAACGC CATGAGTGGA ACAAGCATGG
CTTTTTTCCT AGTATCCCGA GCTGCTTGCC.
```

A further aspect of the invention is directed to a probe selected from synthetic oligonucleotide sequences corresponding to oligonucleotide sequences located within the gp48 nucleotide region of 1362 to 1880 of the BVDV genome. The probe preferably comprises at least 100 nucleotides. In a preferred embodiment of the invention the probe is

4

```
GTCATGAATA GAACCCAAGC CAATCTCACT
GAGGGACAAC CACCAAGGGA GTGCGCAGTC
ACTTGTAGGT ATGATAGGGC TAGTGACTTA
AACGTGGTAA CACAAGCTAG AGATAGCCCC
ACACCCTTAA CAGGTTGCAA GAAAGGAAAG
AACTTCTCCT TTGCAGGGAT ATTGATGCGG
GGCCCCTGCA ACTTTGAAAT AGCTGCAAGT
GATGTATTAT TCAAAGAACA TGAACGGATT
AGTATGTTCC AGGATACCAC TCTTTACCTT
GTTGACGGGT TGACCAACTC CTTAGAAGGT
GCCAGACAAG GA.
```

In the specification and claims the expression "synthetic oligonucleotide sequences corresponding to oligonucleotide sequences located within the nucleotide region" is not intended to be limited to full correspondence, but a few mismatches in the sequences may be acceptable as long as the annealing or hybridization of sequences is adequately specific. For example 5-6 nucleotide mismatches per 20 nucleotides in the sequences should give acceptable results.

Yet another aspect of the invention is directed to a test kit for the detection of an infection caused by BVDV, comprising

a) a first pair of primers according to the invention,

b) optionally a second pair of primers according to the invention, and

c) a probe according to the invention.

The test kit may further contain a buffer of pH 8.3-8.6, each of the four dNTPs, an enzyme, such as Taq-polymerase, and other reagents needed for the use of the kit. In the test kit the probe may be in a hybridization solution or in water. The probe may further be in a labeled form. Advantageously the label is biotin-16-dUTP or digoxigenin-16-dUTP. All the items in the test kit are suitably in separate containers with specific labels. The test kit should be accompanied by written instructions for use.

Short description of the drawings.

Fig. 1 shows the result of a single PCR amplification of BVDV cDNA from cell cultures.

Fig. 2 shows the results of double PCR amplification of BVDV cDNA from specimens of acutely diseased and of persistently infected cattle.

METHODS

Cells and viruses.

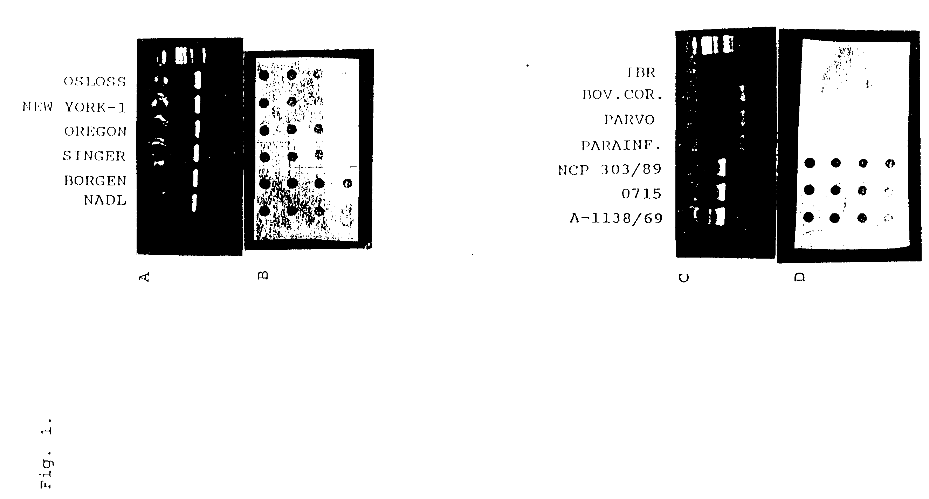

In order to estimate the specificity of the PCR method, we tested various strains of both biotypes of BVDV (kindly provided by Professors B. Liess and V. Moennig, Institute of Virology, Hannover Veterinary School, Hannover, FRG) as well as six local isolates. The viruses were grown on bovine turbinate (BT) cell cultures. Bovine kidney and BT cells inoculated with bovine parvovirus, bovine herpesvirus-1, bovine coronavirus and parainfluenza-3 virus strains were used as controls of specificity (Fig. 1). The cells were harvested when the cytopathic effect (CPE) appeared. The noncp-BVDV strains were harvested after 6 days of incubation. The cells were dispersed by trypsinization, washed two times in PBS and the cell number was estimated.

Clinical specimens.

In order to estimate the diagnostic applicability of the PCR in the disease, organ specimens of acutely diseased calves and serum specimens of persistently infected cattle were tested. The organs were homogenized and 10% v/w suspensions were prepared in PBS. For comparison, the suspensions were tested in parallel by virus isolation and by PCR.

## Conventional diagnostic procedures.

Virus isolation from the specimens was made on BT cells, by performing two passages, 6 days each. The results were read by indirect immunofluorescence, using monoclonal antibodies prepared by Juntti et al. (1987) and by immunoperoxidase method using polyclonal antibodies.

## Preparation of specimens for the PCR.

The specimens were prepared for the PCR as follows. In order to destroy cell membranes the cell and the organ preparates were three times frozen at -20°C. After the last thawing, 1 ml amounts of the specimens were centrifuged in an Eppendorf centrifuge at 500g for 5 minutes. The supernatants were recentrifuged at 5.000g for 15 minutes. The pellets were discarded and the supernatants were pelleted at 50,000 rpm in a Kontron TST-50 rotor for 30 minutes at 15°C. The pellets were collected in TE buffer (10mM Tris pH 7.5 and 1mM EDTA), containing 1.5% SDS. Preinase K (Merck, Darmstadt, FRG) was added to have a final concentration of 100 to 200 µg/ml and the specimens were incubated at 55°C for 30 minutes. Subsequently, they were heated to 95°C for 10 minutes and the 10 to 20 µg fresh yeast RNA (USB Comp., Cleaveland, Ohio, USA) was added as carrier RNA. The samples were extracted twice with fenol/chloroform and precipitated with ethanol. The precipitates were pelleted at 14,000 rpm in an Eppendorf centrifuge for 20 minutes at 4°C. The pellets were dissolved in 10 µl 0.5xTE buffer. In order to perform the reverse transcriptase reaction, the following materials were added to the 10 µl RNA: 2 µl 10xPCR buffer (0.1 M Tris-HCl, pH 8.3; 0.5 M KCl, 25 mM MgCl$_2$, 1 mg/ml BSA), 1 µl of each of the four dNTPs (10 mM stocks, Boehringer, Mannheim, FRG), 2 µl of reverse transcriptase primer (10 µM stock), 1 µl RNAsin (Pharmacia, Uppsala, Sweden), 1 µl reverse transcriptase enzyme (200 U/µl, Moloney MuLV, Bethesda Research Laboratories, Gathersburg, MD, USA). The specimens were allowed to stand at room temperature for 10 minutes, then incubated at 37°C for 90 minutes, subsequently heated to 98°C for 10 minutes and then the cDNA products were chilled on ice.

## Primers and probes.

Six primers were selected, complementary to the published sequences of the gp48 region of the cytopathic NADL virus strain (Collett et al., 1988).

For the identification of the PCR products, three probes were designed. The probes corresponded to various regions between the two internal primers.

The primer sequences, locations and functions as well as the oligonucleotide probe sequences and positions on the BVDV genome are shown in Table 1. The oligonucleotides were synthesized at the Research Genetics, Huntsville, AL, USA. The molecules were purified by reverse phase chromatography. The probes were labeled at the 5′ end with $^{32}$P ATP by using polynucleotide kinase (Pharmacia, Uppsala, Sweden) according to Davis et al. (1986). Biotinylation of oligonucleotide probes was performed at the 3′ end by using the TdT enzyme (Pharmacia, Uppsala, Sweden) in order to add a tail of biotin-16-UTP (Boehringer, Mannheim, FRG), according to Guitteny et al. (1988). In the hybridization assays the oligonucleotide probes were used in a final concentration of 200 ng/ml.

A 310 base pairs (bp) probe, termed pBVDV, was also designed. This probe was synthesized in our laboratory by primer extension (see below).

The first step of amplification (single PCR) was made by applying a single pair of primers, OBVD-5 and OBVD-6. The PCR product was diluted 1 to 1,000 in distilled water and 5 µl was used for the second amplification. Two new primers (OBVD-1 and OBVD-2) were designed to perform the second amplification step. The first and the second PCR were made in 32 cycles each. During the reactions, biotin-16-dUTP (Boehringer, Mannheim, F.R.G.) was incorporated into the newly synthesized molecules. Thus, the biotin-labeling was done directly during the synthesis. The 310 bp probe was purified on G50 columns, and salmon sperm DNA was added to a final concentration of 0.75 mg/ml. Subsequently, the probe was heated to 95°C for 5 minutes and chilled on ice before use. Concentration of the probe was 500 mg/ml in the hybridization mixture.

## The conditions of amplification.

The PCR was made by using 20 µl cDNA (see above), 8µl 10 x PCR buffer (see above) 4 µl of 10 pmole/1 of upstream and downstream BVDV primers, respectively, 0.5 µl (2.5 U) Taq polymerase (AmpliTaq, Perkin-Elmer Cetus, Norwalk, CT, USA), and water to 100 µl. PCR was performed by both a single pair and a double pair of primers (single and double PCR). The single PCR was made in 32 cycles in a DNA Thermal Cycler (Perkin-Elmer Cetus). Each cycle included denaturation at 94°C for 45 seconds, primer annealing at 55°C and primer extension at 72°C for 1 minute each. In the single PCR the primers OBVD-5 and OBVD-6 were used. Double PCR was performed in order to increase the intensity of amplification. In double PCR a pre-amplification was made by using the external primers (OBVD-5 and OBVD-6) at an annealing temperature of 55°C in 32 cycles. Then the second amplification step was performed by adding two internal primers (OBVD-7 and OBVD-8) to the specimens and the reaction was continued at an annealing temperature of 72°C in 32 cycles. The denaturation and the synthesis were made at the same temperatures as above.

### Identification of the PCR products.

To visualize the yield, 10 $\mu$l amounts of the PCR products were run on 2.5% agarose gels at 100 V for 45 minutes. The gels were stained with ethidium bromide as described elsewhere (Maniatis et al., 1982).

To control the specificity of the amplification the PCR products were simultaneously tested by DNA dot-blot hybridization and by Southern-blot hybridization. Filters were hybridized with the $^{32}$P-labeled or with the biotinylated BVDV oligonucleotide probe in the same way as described elsewhere (Davis et al., 1986; Belák et al., 1989; Ballagi-Pordány et al., 1990).

### RESULTS

#### Elctrophoresis of the products of the single PCR.

By applying the single PCR, a product of 520 bp was detected on the gels (Fig. 1). Such a fragment was yielded by each specimen of the cell cultures which had been inoculated with various BVDV strains, but not by specimens of cell cultures infected with heterologous viruses (Fig. 1).

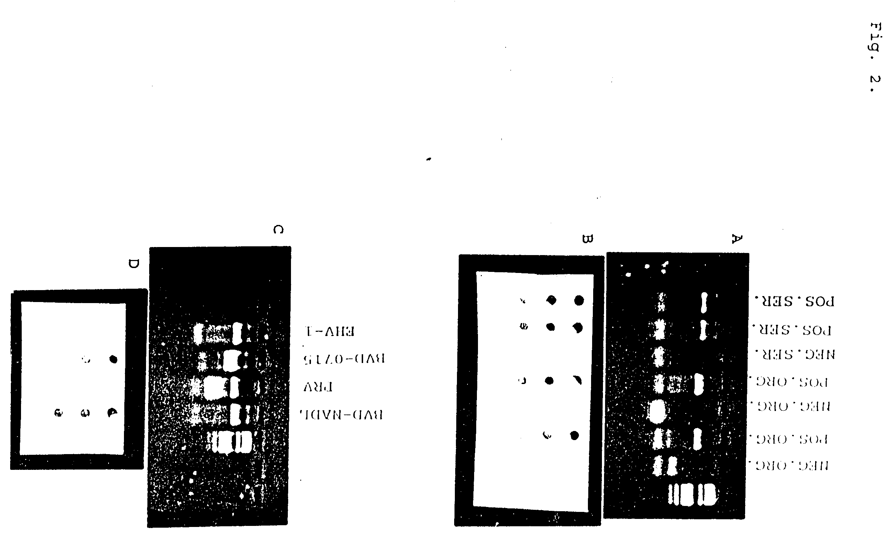

When applying the single PCR, we have been unable to detect the viral RNA in each of the virus-infected organ homogenates tested (not shown). However, in the serum samples of the persistently infected animals the virus was detected even by the single method of amplification (Fig. 2).

#### Electrophoresis of the products of the double PCR.

By applying the double PCR, a band of 432 bp was seen on the gels. By the double amplification the virus signal was detected even in the organ homogenates (Fig. 2).

#### Nucleic acid hybridization of the PCR products.

The Southern blot hybridization as well as the dot-blot hybridization revealed that the bands had nucleic acid sequences specific for BVDV (Figs. 1 and 2). The hybridization studies showed a considerable variability of the various BVDV strains in the regions from where the OBVD-4 and the OBVD-4a probes were synthesized. At 44°C hybridization temperature the OBVD-4 probe recognized two local isolates and the NADL strain only (Table 2). By decreasing the temperature to 21°C, three local isolates, as well as strains NADL, Singer and Borgen were recognized by the probe (not shown). This probe never reacted with the rest of the strains and with three non-cp local isolates. The OBVD-4a probe detected all strains at 44°C, but the intensity of the hybridization signal varied strongly (Table 2). The pBVDV probe recognized each of the strains and showed a hybridization signal of constant intensity (Fig.1 and Table 2).

### Discussion of the results.

The evidence obtained in the present study shows that specific and sensitive PCR assays can be developed to detect BVDV RNA sequences in cell cultures and in clinical material. The experiments show, that due to the heterogenity of the BVDV genome, it is not easy to design a PCR and hybridization system, which is able to recognize all the variants of this virus, thus, may have a safe diagnostic applicability.

The fact, that the 520 bp PCR product was amplified from all of the specimens infected with various BVDV strains indicate that the selection of primers was adequate in these experiments. The primers were also suitable for the detection of the virus from the clinical material. In the serum samples the virus had been detected by single PCR. This indicates that the virus amount was probably higher in the serum samples of persistently infected animals than in the organs of acutely diseased calves. For the detection of the probably few viral copies in the organs, the double PCR provided a sensitive detection system.

In contrast to the primers, the short, 21 bp probes were too specific. They were not sufficient for a wide-range detection of the various strains of BVDV. For example, the OBVD-4 probe recognized only the NADL strain (from which its sequences originated) and two local isolates, S1 and S3. This indicates that in map regions from 1594 to 1616 S1 and S3 are more closely related to the NADL strain than the other isolates. The lack of hybridization to PCR products of the other strains show that OBVD-4 cannot be used as a general probe to detect BVDV infection. The high specificity of OBVD-4 was decreased by applying the lower stringency of 21°C, which allowed five mismatches out of the 21 nucleotides. However, the probe still did not possess the spectrum required for diagnostic use.

The hybridization results of BVDV-4a show that the nucleic acid sequences of this probe are more common in the various BVDV strains. However, the hybridization signals of varying intensity indicate heterologies even in the region of pBVD-4a. This probe can be used in the method of the invention, but for the time being it is not preferred as a general probe.

The problem of too high specificity was solved by applying the long 310 bp probe, which allows more mismatches than the short probes. (It is estimated that a good probe for the purposes of the present invention should comprise at least 100 nucleotides.) The large probe proved to be more practical for additional reasons. The direct incorporation of the biotinylated nucleotide provided a labeling method which was more simple, more effective and more economic than the end labeling of the short probes.

The PCR, together with the simple non-radioactive hybridization assay, proved a novel direct detection method for the BVDV infection. By this method a confirmed direct diagnosis of BVDV infection is made within 48 hours. The results show that the method is an appropriate alternative to the conventional techniques to detect BVDV infection.

## Table 1. Sequences of the synthetic oligonucleotides and their location along the BVDV genome

| Primers* | Sequence | Location and direction |
|---|---|---|
| OBVD 1 | GTCATGAATA GAACCCAAGC C | 1473 - 1493 > |
| OBVD 2 | TCCTTGTCTG GCACCTTCTA A | < 1763 - 1783 |
| OBVD 5 | GGTATGATGG ATGCAAGTGA G | 1362 - 1382 > |
| OBVD 6 | AAGCAGCGTA TGCTCCAAAC C | < 1860 - 1880 |
| OBVD 7 | ACTTCAACGC CATGAGTGGA ACAAGCATGG | 1405 - 1434 |
| OBVD 8 | CTTTTTTCCT AGTATCCCGA GCTGCTTGCC | < 1808 - 1837 |

| Probes | Sequence | Location and direction |
|---|---|---|
| OBVD 4 | ACCCTTAACA GGTTGCAAGA A | 1594 – 1614 > |
| OBVD 4a | GTTCTTTCCT TTCTTGCAAC C | < 1604 – 1624 |
| BVDV | GTCATGAATA GAACCCAAGC CAATCTCACT GAGGGACAAC CACCAAGGGA GTGCGCAGTC ACTTGTAGGT ATGATAGGGC TAGTGACTTA AACGTGGTAA CACAAGCTAG AGATAGCCCC ACACCCTTAA CAGGTTGCAA GAAAGGAAAG AACTTCTCCT TTGCAGGGAT ATTGATGCGG GGCCCCTGCA ACTTTGAAAT AGCTGCAAGT GATGTATTAT TCAAAGAACA TGAACGGATT AGTATGTTCC AGGATACCAC TCTTTACCTT GTTGACGGGT TGACCAACTC CTTAGAAGGT GCCAGACAAG GA. | 1473 – 1783 |

*Functions of primers:

OBVD 1 and OBVD 2: primers for synthesis of the large probe pBVD

OBVD 5 and OBVD 6: external primers

OBVD 7 and OBVD 8: internal primers

OBVD 6 : also used as reverse transcriptase primer

Table 2. Detection range of the probes. Single PCR was performed with cell culture specimens inoculated with various virus strains. When tested in electrophoresis, in each The PCR products were blotted and hybridized at 44°C with the various probes.

| Virus strains and isolates | Cp effect | Hybridization with probe | | |
|---|---|---|---|---|
| | | A | B | C |
| A-1138/69 | + | NT | NT | + |
| NADL | + | + | + | + |
| New York | - | - | +* | + |
| OregonC24V | + | NT | NT | + |
| Osloss2482 | + | NT | NT | + |
| Singer | + | - | +* | + |
| Ug59 | + | - | + | + |
| 0715/80 | - | NT | NT | + |
| S1** | - | + | + | + |
| S2 | - | - | +* | + |
| S3 | - | + | + | + |
| S4 | - | - | + | + |
| S5 | - | - | +* | + |
| S6 | - | - | + | + |

A, probe OBVD-4; B, probe OBVD-4a; C, probe pBVDV

* weak hybridization signal

** Swedish isolates

10

Table 3. Detection of BVDV genomic sequences in clinical specimens. Number of positive cases versus total number of specimens examined by virus isolation and by the PCR.

| Specimens from cows | Virus isolation | PCR single | PCR double |
|---|---|---|---|
| From acute cases | | | |
| lungs | 1/4 | 0/4 | 2/4 |
| spleen | 2/5 | 0/5 | 2/5 |
| placenta | 0/2 | 0/2 | 0/2 |
| From chronic cases | | | |
| serum | 5/8 | 4/8 | 5/8 |
| total | 8/19 | 4/19 | 9/19 |

Detailed description of the drawings.

Fig 1. Single PCR amplification of BVDV cDNA from cell cultures. A and C, electrophoresis of the PCR products of cell infected with BVDV strains NADL (A1), Ug59 (A2), Singer (A3), Oregon (A4), New York (A5), Osloss (A6), A-1138/69 (C1), 0175 (C2) and 303/89 (C3). PCR products from cells infected with parainfluenza-3 virus (C4), parvovirus (C5), bovine coronavirus (C6) and bovine herpesvirus-1 (C7). DNA size marker $\Phi$ X-174-RF/HaeIII digest is seen in positions A7 and C8. B and D, dot blot hybridization of the same cell culture samples with the pBVDV probe.

Fig. 2. Double PCR amplification of BVDV cDNA from specimens of acutely diseased and of persistently infected cattle. A, electrophoresis of the PCR products of serum specimens of persistently infected cattle positive in virus isolation (1 and 2) and negative in virus isolation (3). Organ homogenates of acutely infected cattle positive (4 and 6) and negative (5 and 7) in virus isolation. DNA size marker in position 8 is the same as in Fig. 1.

REFERENCES

BAKER, J. C. (1987). Bovine viral diarrhea virus: A review. Journal of American Veterinary Medical Association 190, 1449-1458.

BALLAGI-PORDANY, A., KLINGEBORN, B., FLENSBURG, B. & BELAK, S. (1990). Equine herpesvirus type 1: Detection of viral DNA sequences in aborted fetuses with the polymerase chain reaction. Veterinary Microbiology, in press.

BELAK, S., BALLAGI-PORDANY, A., FENSBURG, J. & VIRTANEN, A. (1989). Detection of pseudorabies virus DNA sequences by the polymerase chain reaction. Archives of Virology 108, 279-286.

BOLIN, S. R., McCLURKIN, A. W., CUTLIP, R. C. & CORIA, M. F. (1985). Response of cattle persistently infected with noncytopathic bovine viral diarrhea virus to vaccination for bovine viral diarrhea and to subsequent challenge exposure with cytopathic bovine viral diarrhea virus. American Journal of Veterinary Research 46, 2476-2470.

BROWNLIE, J., CLARKE, M. C. & HOWARD, C. J. (1984). Experimental production of fetal mucosal disease in cattle. Veterinary Record 114, 535-536.

COLLETT, M. S., LARSON, R., GOLD, C., STRICK, D., ANDERSON, D. K. & PURCHIO, A. F. (1988). Molecular cloning and nucleotide sequence of the pestivirus bovine viral diarrhea virus. Virology 165, 191-199.

DAVIS, L. G., DIBNER, M. D. & BATTEY, J. F. (1986). Basic methods in molecular biology. Elsevier, New York.

DIDERHOLM, H. & DINTER, Z. (1966). Infectious RNA derived from bovine viral diarrhea virus. Zentralblatt fur Bacteriologie I. Abt. Orig. 201, 270-272.

GUITTENY, A. F., Fouque, B., Mougin, C., Teoule, R. & Bloch, B., (1988). Histological detection of messenger RNAs with biotinylated synthetic oligonucleotide probes. Journal of Histochemistry and Cytochemistry, 36, 563-571.

JUNTTI, N., LARSSON, B. & FOSSUM, C. (1987). The use of monoclonal antibodies in enzyme linked immunoadsorbent assay for detection of antibodies to bovine viral diarrhoea virus. Journal of Veterinary Medicine 34, 356-363.

MANIATIS, T., FRITSCH, E.F. & SAMBROOK, J. (1982). Molecular cloning. A laboratory manual. Cold Spring Harbor Laboartory, Cold Spring Harbor, New York.

POTGIETER, L. N. D. & BROCK, K. V. (1989). Detection of bovine viral diarrhea virus by spot hybridization with probes prepared from cloned cDNA sequences. Journal of Veterinary Diagnostic Investigation, 1, 29-33.

PURCHIO, A. F., LARSON, R. & COLLETT, M. S. (1983). Characterization of virus-specific RNA synthesized in bovine cells infected with bovine viral diarrhea virus. Journal of Virology 48, 320-324.

RENARD, A., GUIOT, C., SCHMETZ, D., DAGENAIS, L., PASTORET, P. P., DINA, D. & MARTIAL, J. A. (1985). Molecular cloning of bovine viral diarrhea viral sequences. DNA 4, 429-438.

WESTAWAY, E.G., BRINTON, M. A., GAIDAMOVICH, S. Ya., HORZINEK, M. C., IGARASHI, A., KÄÄRIÄINEN, L., LVOV, D. K., PORTERFIELD, J. S., RUSSELL, P. K. & TRENT D. W. (1985). Togaviridae. Intervirology, 24, 125-139.

## Claims

1. A method of detecting an infection caused by a specific type of virus in a biological specimen, whereby said specimen is treated in a per se known manner for the purification of viral RNA and transcription of a selected RNA sequence to the corresponding cDNA, followed by a first amplification of said cDNA by the polymeraee chain reaction (PCR) using a first pair of primers, optionally dilution of the PCR product and optionally a second amplification of said cDNA by PCR, then said type of virus is identified by hybridizing said amplified cDNA with a labeled probe specific for said type of virus and possibly formed hybrides are detected with the aid of the label used, characterized in that the specific type of virus is bovine viral diarrhea virus (BVDV) and that the first pair of primers, the optional second pair of primers and the probe are all selected from synthetic oligonucleotide sequences corresponding to oligonucleotide sequences located within the gp48 nucleotide region of 1362 to 1880 of the BVDV genome.

2. A method according to claim 1, characterized in that the first pair of primers consist of 19 to 23 nucleotidee each, the second pair of primers consist of 27 to 32 nucleotides each and that the number of nucleotides in each of the second pair of primers is at least 8 more than in each of the first pair of primers.

3. A method according to claim 2, characterized in that the primers are selected from the group consisting of

```
GGTATGATGG ATGCAAGTGA G
AAGCAGCGTA TGCTCCAAAC C
ACTTCAACGC CATGAGTGGA ACAAGCATGG
CTTTTTTCCT AGTATCCCGA GCTGCTTGCC.
```

4. A method according to anyone of claims 1-3, characterized in that the probe is

```
GTCATGAATA GAACCCAAGC CAATCTCACT
GAGGGACAAC CACCAAGGGA GTGCGCAGTC
ACTTGTAGGT ATGATAGGGC TAGTGACTTA
AACGTGGTAA CACAAGCTAG AGATAGCCCC
ACACCCTTAA CAGGTTGCAA GAAAGGAAAG
AACTTCTCCT TTGCAGGGAT ATTGATGCGG
GGCCCCTGCA ACTTTGAAAT AGCTGCAAGT
GATGTATTAT TCAAAGAACA TGAACGGATT
AGTATGTTCC AGGATACCAC TCTTTACCTT
GTTGACGGGT TGACCAACTC CTTAGAAGGT
GCCAGACAAG GA.
```

5. A primer selected from synthetic oligonucleotide sequences corresponding to oligonucleotide sequences located within the gp48 nucleotide region of 1362 to 1880 of the BVDV genome.

6. A primer according to claim 5, characterized in that it is selected from primers consisting of 19 to 32 nucleotides.

7. A primer according to claim 6, characterized in that it is selected from the group consisting of

```
GGTATGATGG ATGCAAGTGA G
AAGCAGCGTA TGCTCCAAAC C
ACTTCAACGC CATGAGTGGA ACAAGCATGG
CTTTTTTCCT AGTATCCCGA GCTGCTTGCC.
```

8. A probe selected from synthetic oligonucleotide sequences corresponding to oligonucleotide sequences located within the gp48 nucleotide region of 1362 to 1880 of the BVDV genome.

9. A probe according to claim 8, characterized in that it is

```
GTCATGAATA GAACCCAAGC CAATCTCACT
GAGGGACAAC CACCAAGGGA GTGCGCAGTC
ACTTGTAGGT ATGATAGGGC TAGTGACTTA
AACGTGGTAA CACAAGCTAG AGATAGCCCC
ACACCCTTAA CAGGTTGCAA GAAAGGAAAG
AACTTCTCCT TTGCAGGGAT ATTGATGCGG
GGCCCCTGCA ACTTTGAAAT AGCTGCAAGT
GATGTATTAT TCAAAGAACA TGAACGGATT
AGTATGTTCC AGGATACCAC TCTTTACCTT
GTTGACGGGT TGACCAACTC CTTAGAAGGT
GCCAGACAAG GA.
```

10. A test kit for the detection of an infection caused by BVDV, comprising
   a) a first pair of primers according to anyone of claims 5-7,
   b) optionally a second pair of primers according to anyone of claims 5-7, and
   c) a probe according to anyone of claims 8 or 9.

Fig. 1.

Fig. 2.

| European Patent Office | EUROPEAN SEARCH REPORT | Application Number |
|---|---|---|

EP  91 85 0139

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,A | CHEMICAL ABSTRACTS, vol. 113, no. 17, October 1990, page 222, abstract no. 146691z, Columbus, Ohio, US; B.A. SCHROEDER et al.: "Specific sequence amplification of bovine viral diarrhea virus nucleic acid", & ARCH. VIROL. 1990, 111(3-4), 239-46 * Abstract * | 1 | C 12 Q    1/70<br>C 12 Q    1/68<br>C 07 H   21/04   //<br>C 12 P   19/34 |
| A | CHEMICAL ABSTRACTS, vol. 110, no. 1, 2nd January 1989, page 204, abstract no. 2134k, Columbus, Ohio, US; K.V. BROCK et al.: "Molecular cloning of complementary DNA from a pneumopathic strain of bovine viral diarrhea virus and its diagnostic application", & CAN. J. VET. RES. 1988, 52(4), 451-7 * Abstract * | 1-4,8,9 | |
| P,A | EP-A-0 403 384  (EUROGENTEC et al.) * Whole document * | 1-4,8-9 | TECHNICAL FIELDS SEARCHED (Int. Cl.5) |
| P,A | BIOLOGICAL ABSTRACTS, vol. 91, no. 9, 15th May 1991, abstract no. 97196, Philadelphia, PA, US; C. HERTIG et al.: "Detection of bovine viral diarrhea (BVD) virus using the polymerase chain reaction", & VET. MICROBIOL. 26(1/2): 65-76. 1991 * Abstract * | 1-4,8-9 | C 12 Q<br>C 12 N<br>C 07 K<br>A 61 K |

The present search report has been drawn up for all claims

| Place of search | Date of completion of the search | Examiner |
|---|---|---|
| THE HAGUE | 04-10-1991 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document